# EUROPEAN PATENT APPLICATION

(11) **EP 3 731 232 A1**
(43) Date of publication of application: **28.10.2020**
(21) Application number: 20170333.7
(22) Date of filing: 20.04.2020
(51) Int. Cl.: G16H 10/40, G16H 40/67, C12M 1/00, H04L 9/32

(54) **RESEARCH DATABASE**

(30) Priority: 26.04.2019 NO 20190555
(71) Applicant: Gudesen, Hans Gude, 1639 Gamle Fredrikstad (NO)
(72) Inventor: Gudesen, Hans Gude, 1639 Gamle Fredrikstad (NO)
(74) Representative: Acapo AS

(57) **Abstract**

A biobank and database system for storage of and interactions with biological samples in vitro is disclosed. The system comprises incubators for living tissue, and a number of biological samples where each biological sample is either an organoid or a sample containing live tissue, supported by or containing cellulose derived from tunicates. The system further comprises means for monitoring and manipulating each biological sample, a chronologic ledger with automatic registration of all relevant data pertaining to provenance and pre-history of each biological sample as well as information on all manipulations performed on each biological sample and results obtained. The system further comprises one or more nodes arranged to communicate with the incubators, and control access to the chronologic ledger and to the biological samples in the incubators. The chronologic ledger may be structured as a blockchain.

## Description

### Field of the invention

The present invention generally relates to research on living tissue in vitro.

### Background of the invention.

Research on living tissue in vitro is expanding rapidly, linked to drug development and testing, gene therapy, regenerative medicine and tissue engineering (RTE), cosmetic procedures and basic science. During the last few years, great strides have been taken within these fields, supported by developments in, e.g., immune response control, stem cell technologies, novel biocompatible materials and advanced tissue test platforms, in particular 3D printing of mini-organs and organoids.

According to Wikipedia (https://en.wikipedia.org/wiki/Organoid), an **organoid** is a miniaturized and simplified version of an organ produced in vitro in three dimensions that shows realistic micro-anatomy. Organoids are derived from one or a few cells from a tissue, embryonic or adult stem cells or induced pluripotent stem cells, which can self-organize in three-dimensional culture owing to their self-renewal and differentiation capacities. The technique for growing organoids has rapidly improved since the early 2010s, and it was named by *The Scientist* as one of the biggest scientific advancements of 2013. Organoids are used by scientists to study disease and treatments in a laboratory to improve diagnostics and therapy, but the scope of applications is much wider, including drug development, testing of food for additives and harmful substances, detection of counterfeit medicines, regenerative medicine and tissue engineering (RTE).

Although very small (typical diameter from 100 micrometer to 5 mm) when compared to the full size organs, organoids self-organize into replications of much of the complexity of an organ. They are still crude mimics of their life-sized counterparts, but the process aims at replicating more and more of the functionality of a human organ. The ultimate goal is to learn how to grow full size organs, from a person's own cells or from superdonor stem cell lines. Potentially there are as many types of organoids as there are tissues and organs in the body. Organoids that are mini-copies of the major organs, like heart, brain, kidney, intestine, stomach, liver, lung, pancreas, have already been tested by different research groups. This way of culturing tissues and mini-organs displays details of how organs and tissue form and grow, and provides new insights on human development and diseases as well as the opportunity to see how e.g. drugs interact with these organoids, potentially revolutionizing the field of drug discovery and opening new approaches to personalized medicine. Human organoid and disease models make it possible to compare natural physiological versus pathological/diseased state response. Cell growth and differentiation pattern of tumor cells versus healthy cells in organoid models can highlight discriminating markers that can subsequently be used as drug targets.

Very large resources are now being expended worldwide to convert the potential represented by these various fields of research into procedures, medicines and materials that can have a decisive impact on the health and well-being of the population in general. The following themes are of particular relevance in the present context:

### Drug discovery

Employing ex vivo tissue technologies to enable massively parallel, high throughput tests of efficacy and safety of new drugs on tissues and organs that represent closely the in vivo counterparts, saving time and resources and reducing the need for animal testing.

### Personalized medicine:

Individual testing and treatment based on harvested tissue from each patient provides an avenue for focused medical procedures without the danger of rejection by the immune system. Stem cells are central in this context. Following upon the discovery of induced pluripotent stem cells (iPSC), it is now possible to establish biobanks of personalized stem cell material for all persons alive, whereas it previously could only be obtained from embryonic cells. There are a growing number of stem cell (bio)banks (> 550 banks as per 2018), offering specialised, commercial processing and long term storage of such stem cell material.

### Regenerative medicine- tissue engineering (RTE):

This field is about developing and implementing replacements for living, functional tissues and organs damaged by age, disease, congenital defects, unhealthy lifestyles or accidents. It is also about stimulating previously irreparable organs and other body parts (i.e., bone, cartilage, blood vessels, bladder, skin) to heal themselves. Importantly, the organs being created have the potential to address the problems related to the shortage of organs available through donation, compared to the number of patients that require life-saving organ transplantation. It is estimated that as many as one in three of Americans and Europeans could benefit from **RTE** within the next 10 years. Examples covered by **RTE** also include cell therapies (the injection of stem cells or progenitor cells) and immunomodulation therapy (regeneration by biologically active molecules administered alone or as secretions by infused cells).

Progress within these fields shall be critically dependent upon availability of technical infrastructure and enabling technologies, in particular the following:

### 3D bioprinting with stem cells:

A living tissue sample is harvested from a person or animal. The sample is used to provide viable cell material for creating a line of stem cells, which are mixed with scaffolding material and growth factors (diffusible signaling proteins that stimulate cell growth, differentiation, survival, inflammation, and tissue repair) into a bio-ink and then printed out as living 3D organoids, i.e. individualized, complex collections of cells that are representations of a person's organs and tissues, to be used for R&D, diagnostics, therapeutics, drug screening and testing, etc. Ultimately, 3D bioprinting shall enable the generation of precisely controlled 3D cell models and tissue constructs, by engineering anatomically-shaped substrates with tissue-like complexity. As stem cells proliferate on a structural backbone formed by scaffolding materials in the ink, they reconstruct this tissue into a functional organ. The stem cells are critical parts of the overall 3D bioprinting/bioink/**RTE** concept. Until 2006 stem cells could only be acquired from embryonic cells, which are naturally so called pluripotent (cells that can give rise to all of the cell types that make up the body). In that year Shinya Yamanaka found a new way to reprogramme adult, specialized cells into "induced pluripotent stem cells" (iPSC). His discovery means that theoretically any dividing cell of the body can now be turned into a pluripotent stem cell. He achieved this by adding four genes to skin cells, thereby starting a process inside the cells called reprogramming. Within just a few days, a specialised cell is then returned back to the starting point, the embryonic, or pluripotent state, from which a cell can develop into any differentiated state. This is like going back in time, to the embryo level, and then, forward in time, into any type of differentiated, specialised cells. This can be done with all cells from all persons alive. 3D bioprinted organs with such pluripotent cells will not be rejected by the body of the person from whom the cells where taken. The bioprinted cells will not be identified as strangers or threats, not be recognised by the person's immune system. iPSCs are, however, not perfectly understood yet, and it will take time to achieve the goal of making artificial 3D full size organs.

### Reactor/incubation systems:

There are currently a number of institutions worldwide that are engaged in running biobanks where living tissue samples are incubated and studied on a large scale. As an example, "Foundation Hubrecht" in the Netherlands operate their "Organoid Biobank" consisting of a variety of organs and disease models (http://hub4organoids.eu/living-biobanks/): "HUB Biobank Database is an initiative of HUB and the Cancer Genomics Centre in Netherlands. It consists of a catalog of all models that are present in the HUB Organoid Biobank. Furthermore, the extensive characterization that has been done for each of the models can be found here. This characterization consists of genetics, histological and clinical data accompanied by responses to drug treatments relevant for the disease model. The HUB Organoid Database contain the models of the intestine, liver, pancreas, lung, mammary gland, and others. Diseases such as Cancer, Cystic Fibrosis, IBD, etc are available for the relevant organs. The Organoid biobank is a powerful resource for many aspects of Biomedical research such as target identification, drug screening and development. Because the HUB Organoid Biobank embodies the heterogeneity of the patient population, it provides a preclinical method to obtain insights into drug responses that would otherwise only become visible during or even after clinical trials on patients". Foundation Hubrecht work with Stemcell Technologies to produce organoids that are distributed to scientists worldwide. Other groups in Europe and the US are also involved in similar organoid-banking services.

### Robotics:

Accommodation and handling of vast numbers of tissue samples in biobanks is extremely challenging and highly multidisciplinary, and requires the highest levels of control and precision. In practice, cost effective, large scale operations must imply the use of robots. An example of how this can be implemented has been demonstrated in work performed at the University of Washington (Health Sciences/UW Medicine): "Robots grow mini-organs from human stem cells: Robotic approach could accelerate regenerative medicine research and drug discovery." ScienceDaily, 17 May 2018. <www.sciencedaily.com/releases/2018/05/180517123300.htm>.

### Data security:

Data integrity and privacy issues are of paramount importance when handling medical and other types of personal data from a population. Depending on circumstances, many types of access control and logging systems have been implemented in hospitals and other institutions. Blockchains are used in cases where tamper-free chronologic records are important and are well known in connection with financial transactions and corporate research tracking. Recently, systems employing block-chain principles have been introduced in the medical sector, cf., e.g.: C.T. McFarlane "Blockchain network for secure exchange of healthcare information"; US2019027237 (A1), and D.A. Bulleit et al.: "Blockchain-based mechanisms for secure health information resource exchange"; WO2018039312 (A1).

Thus, it would seem that mankind now stands at the cusp of a new era in medical science, enabled by recent developments in a number of key disciplines. However, certain serious obstacles, technical and legal as well as structural, must be overcome before significant progress can be made beyond the current situation. Prominent amongst these are the following:
- Biobanks are central to all research referred above. Presently they are run in closely guarded, proprietary environments under narrow and specialized objectives, e.g. embryonic stem cell storage in a strictly commercial setting. What is needed is a generalized biobank system that can be made accessible to a global community of researchers and institutions, in a way that promotes scientific progress on a broad front and at the same time safeguards the interests of all participants.
- Regardless of how the biobanks are organized and operated, security and protection of personal data is of paramount importance and must be integrated into any system where information about biological samples is stored and retrieved. Responsibility for overseeing these aspects of data flow must be vested with appropriate institutional authorities.
- Priority and peer recognition is a strong driving force for researchers and has traditionally been taken care of by publication in recognized scientific journals. This solution is in the process of becoming obsolete in light of developments driven by instant electronic communications. It is necessary to establish novel procedures for establishing priority and ownership to scientific results where interactions via biobanks constitute the primary research and dissemination vehicles.
- Ownership to research results and protection of intellectual property rights (IPR) in general are basic prerequisites for organizations who engage in commercialization of biomedical R&D. As a rule, it is desirable to restrict access to sensitive information from the public domain until IPR protection has been obtained, typically in the form of patents. This must be taken into account in any widely used system for sharing of research results. Thus, there is a need to resolve as far as possible the conflict between the need for secrecy on the one hand and the need for efficient and fast communication between research groups on the other hand.
- Any system that collects and stores research data on an interactive basis between researchers and institutions must operate under a common set of procedures and rules that ensure transparency and consistency. Thus, there is currently a need for systems where an authority or gatekeeper oversees that all users of the system adhere to a consensus protocol which regulates issues such as access control, priority, standardization and nomenclature.
- Ultimately, construction of living tissue in vitro shall depend on the materials used. Despite great efforts by the worldwide medical research community, it remains a fact that at present most biomaterials currently being tested in reconstructive medicine and tissue engineering fall far short of being suitable for large scale clinical use. One of the most urgent of the unsolved problems is related to the need for new materials that can function as scaffolding of the organoids and organs in question. These materials must provide sufficient strength and durability, without causing a negative immune system response and subsequent damage, and ultimately degradation and dismantling of the scaffolding material.
- Robotics, automation and remote operations are absolute prerequisites for large scale processing of samples in interactive, global networks of biobanks. Currently, there are efforts on isolated aspects of these aspects, but integrated systems are lacking.

### Summary of the invention.

The objectives are achieved according to the invention by a biobank and database system as defined in claim 1 and a method for operating the biobank and database system as defined in claim 16.

A number of non-exhaustive embodiments, variants or alternatives of the invention are defined by the dependent claims.

A first aspect of the invention is a biobank and database system for storage of and interactions with biological samples in vitro, comprising:
- incubators for living tissue;
- a number of biological samples, where each biological sample is either an organoid or a sample containing live tissue, and supported by or containing cellulose derived from tunicates, and arranged in one or more of the incubators;
- means for monitoring each biological sample;
- means for manipulating each biological sample;
- a chronologic ledger with automatic registration of data pertaining to:
   provenance and pre-history of each biological sample, including materials and procedures employed in extraction and preparation,
   manipulations performed on each biological sample, and
   results obtained;
- one or more nodes arranged to communicate with the incubators, and control access to the chronologic ledger and to the biological samples in the incubators, each node comprising a logic unit, a data storage facility, and a communication interface.

Optionally, the cellulose derived from tunicates is tunicate nanocellulose.

Optionally, the biological samples in the incubators are formed by one or more of the following techniques: 3D bioprinting, micro-contact printing, nano-imprint lithography, selective etching and electrospinning.

Optionally, the means for manipulating are robotic.

Optionally, the chronologic ledger is arranged for performing automatic registration of accesses to at least one of the biological samples, including accesses for purely monitoring purposes. Even further, the chronologic ledger can be searchable.

Optionally, two or more of the nodes form a communicating network operated under a consensus protocol, and optionally, the communicating network is only accessible to a limited set of participants that form a consortium.

Optionally, subsets of consortium participants can be formed when conforming with the consensus protocol.

Optionally, each node in the consortium is arranged for regulating network access for individual research entities associated with that node.

Optionally, the chronologic ledger is arranged for being globally accessible for searches, including to parties outside the consortium, under a protocol where each search query incurs a fee.

Optionally, the chronologic ledger is structured as a blockchain.

Optionally, individual biological samples are monitored by microsensors that transmit data to the chronologic ledger.

Optionally, the biobank and database system is operated in an autonomous or semi-autonomous mode under Artificial Intelligence (Al) guidance.

Optionally, the biobank and database system is operated for screening tests of at least one from the group comprising: medicines, allergens, toxic chemicals, growth factors, chemical additives in foodstuff or consumer products.

A second aspect of the invention is a method for operating a biobank and database system, where the method comprises at least one the following steps:
- i) depositing one or more organoids and/or tissue samples that are supported by or contain cellulose derived from tunicates, at specific locations in one or more incubators;
- ii) bringing the organoids and/or tissue samples in contact with a test substance;
- iii) monitoring the organoids and/or tissue samples; and
- iv) recording data from events related to the organoids and/or tissue samples in a chronologic ledger organized as a blockchain.

Optionally, the test substance is a foodstuff or a therapeutic substance.

Optionally, the deposition of one or more organoids and/or tissue samples is performed by 3D printing or by transporting a pre-made organoid or tissue sample to the biobank.

### Short description of the figures.

Embodiments of the present invention will now be described, by way of example only, with reference to the following diagrams wherein:
Figure 1 discloses the basic units in a biobank and database system comprising a single node with blockchain stored data, a single operator and a single incubator.
Figure 2 discloses a network of nodes that form a distributed peer-to-peer blockchain system with multiple nodes, operators and incubators, at an early stage with only one sample in one incubator.
Figure 3 discloses a network of nodes that form a distributed peer-to-peer blockchain system with multiple nodes, operators and incubators, at a later stage with separate samples in two incubators.

### Detailed description of embodiments of the invention.

### The concept.

A biobank and database system as taught in the present invention is an incubated collection of biological tissues derived from humans or other life forms and subjected to specific procedures that are recorded in a tamper-free, searchable ledger system. The biobank and database systems are used to create new knowledge about healing and growth processes and can provide biomaterials for drug and toxicity testing, regenerative medicine and tissue engineering **(RTE**). Ultimately, human organs individually adapted for each person in question may be prepared and stored in biobank and database systems, in readiness for transplantation as needed.

According to the present invention, the biobank and database system is enabled by the combined application of materials, equipment and procedures as follows:

### Organoid formation using tunicate nanocellulose scaffolding materials:

The microenvironment of the organoids in the incubators is critical. The extracellular matrix (ECM) regulates growth and differentiation via a complex array of signaling mechanisms which are mechanical as well as chemical in nature. In order to simulate the native ECM of a stem cell niche or mature tissue, it is generally required to provide a 3D scaffold with relevant microscale or nanoscale topography. Many techniques and materials are being tested for creating organoids, depending on their intended use: Among the most prominent are 3D bioprinting techniques, but other additive, moulding/stamping or subtractive techniques are being investigated, among them micro-contact printing, nano-imprint lithography, selective etching and electrospinning. Cf., e.g.: X. Tin et al.: "Engineering Stem Cell Organoids", Cell Stem Cell 18, 25-38, January 7, 2016.

A major obstacle to bioengineering of viable organoids has been the requirement of a suitable structural material that can form a scaffold or backbone for true 3D structures, different from the 2D structures used in the state-of-the-art, organ-on-chips ("OOC") approach. Potential scaffolding materials that have been tried include alginate, collagen, chitosan, gelatin, fibrin, wood cellulose, and bacterial cellulose . None of these have proven satisfactory, the main reasons being rejection response due to bio-incompatibility and endotoxins as well as instability, degradation, lack of mechanical integrity, and price. Nanocellulose isolated from tunicates represents a new and radically improved type of scaffolding material.Tunicates are a diverse group of marine filter-feeding animals that have both benthic (living on attached substrates) and pelagic (living in the water column) groups. They are the only animals that can synthesize cellulose, which is different from plant/wood cellulose. Tunicate cellulose fibrils are longer, thicker and have a much larger surface area. These are important structural traits that make composites of tunicate cellulose substantially stronger and will enable a very robust binding with other materials to its surface. Another important advantage is that since tunicates are supported by surrounding seawater, they do not need the same structural support as plants that remain upright on land. The structural matrix is therefore absent of cross-linking lignin and hemicellulose. This means that tunicate cellulose can easily be extracted with >99.7% purity. Stable dispersions of pure tunicate nanocellulose with very low endotoxin content are now commercially available, exhibiting unique viscoelastic properties that make them very suitable in bioinks for 3D Bioprinting, either alone or mixed in with one or more other types of scaffolding materials such as alginates.

### Reactor/incubation system with secure sample handling and ledger function:

Each organoid is stored in an incubator and assigned a unique address, coupled with information about provenance and pre-history. All interactions with a given organoid in the biobank and database system take place by robotic actuators under remote control, and every access to a given organoid is automatically recorded in an electronic ledger system. A single harvest of cell material from a given donor may give rise to a large number of organoids that are all similar but with individual identities. Individual organoids can be accessed for specific interactions, either invasive or non-invasive. In all cases, the results of each interaction is stored in the chronological ledger for that specific organoid. Some of these interactions will permanently modify the virgin organoids, and some will also require longterm testing. Accordingly, there will have to be created a new occupancy in the biobank and database system with an additional organoid each time a fully virgin state is of essence. In this way, the cell material from one single individual may be distributed between a growing number of occupancies in the biobank and database system.

Blockchain protection: According to the present invention, each ledger in the biobank and database system is structured as a blockchain, protecting the stored data from retroactive changes and preserving a true record of who did what/when and the results obtained. Blockchains may be set up in various ways, cf. below. Access to any given blockchain shall be related to either a physical monitoring of or action on the organoid in question or a reading of the contents in the blockchain. All accesses shall be subject to security protocols, typically administered by a computer that hosts the blockchain data in question and/or a computer associated with the incubator where the organoid resides. Access events may typically be recorded as a blockchain element. In general, the biobank and database systems shall be locally operated and globally accessible for personnel within a community of authorized users.

To assist in the understanding of the present invention, some preferred embodiments shall now be described in more detail:

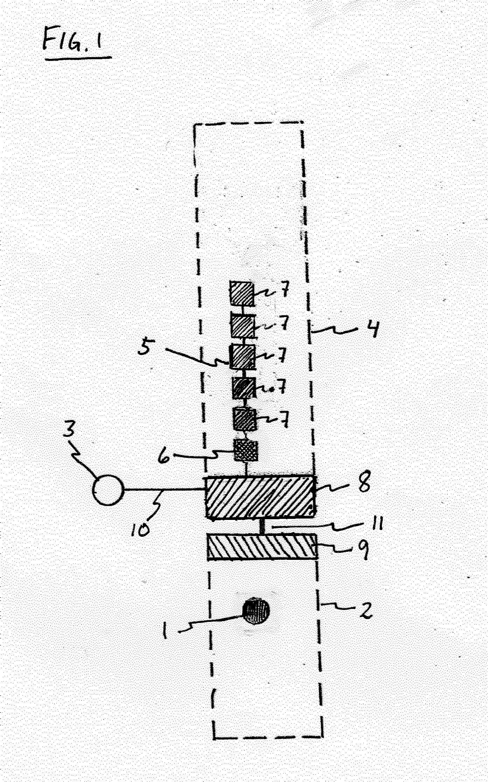

**Fig.1** shows the basic units in a biobank and database system: A sample (**1**) of the biological material is maintained in an incubator (**2**), where it can be subjected to various forms of treatment and analysis according to instructions from an operator (**3**). Each step of the interaction with the sample is recorded in a chronologic ledger in a **node (4).** The node controls and regulates access to the chronologic ledger and to the organoids and tissue samples in the incubator and includes a logic unit, a data storage facility, and a communication interface, The logic system and a data storage facility are operated under a set of security protocols. An essential feature is that the identity and complete history of the sample (**1**), including timestamps, is stored as a blockchain (**5**). As is well known, a blockchain can provide protection against tampering with the stored data and functions as a complete and sequential ledger of events (cf., e.g.: https://en.wikipedia.org/wiki/Blockchain). The record of interactions with the sample starts with a genesis block (**6**) which contains information about the sample provenance and location as well as all relevant parameters defining the initial state of the sample. Every event thereafter affecting the sample is then encapsulated in an information package, a block (**7**), which is added to the previous block in the chain according to a security protocol involving encryption of the block contents. Thus is formed a chain which cannot be altered retroactively but which allows the addition of new blocks to the existing chain by qualified operators employing the appropriate encryption.

In order to perform research on the sample (**1**), an operator (**3**), e.g. a biomedical researcher at an accredited research center, contacts the node (**4**) via a secure line (**10**) to the node interface (**8**) which controls access to the node data system and communication with the incubator interface (**9**). The physical location of the incubator is typically at a distance from the node data system, which implies that the communication between the node and incubator interfaces shall include encryption and access control. If the operator is granted access to the incubator, the operator sends a message about the actions to be performed on the sample (**1**) to the node interface (**8**), which transmits the message to the incubator interface (**9**). At the incubator, a sequence of actions reflecting the message sent from the operator are carried through. All data pertaining to these actions are transmitted to the node, where the logic system creates an information package and adds a block to the blockchain.

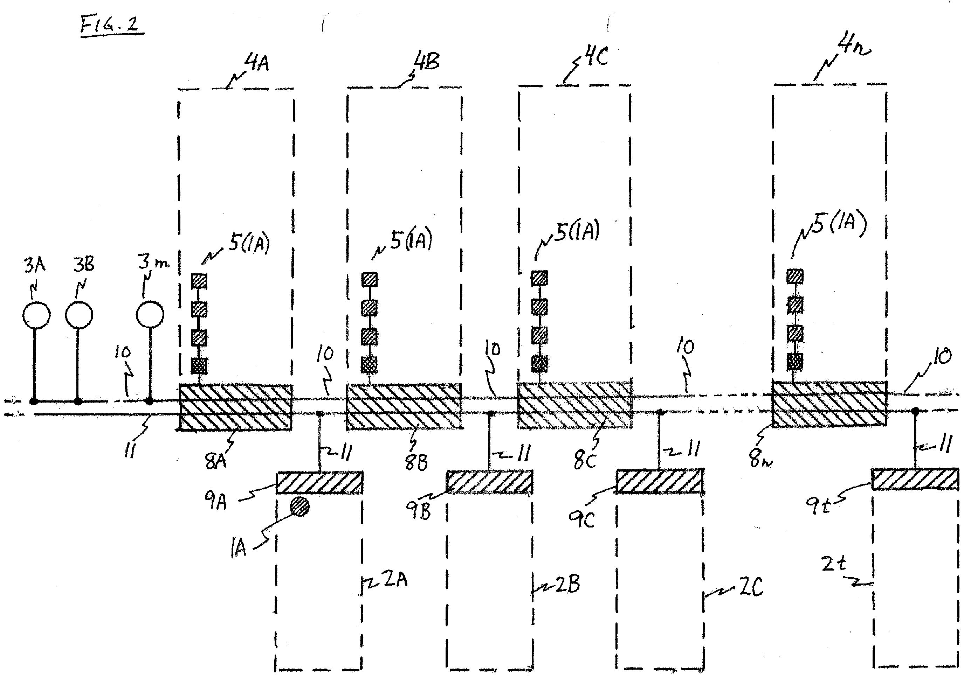

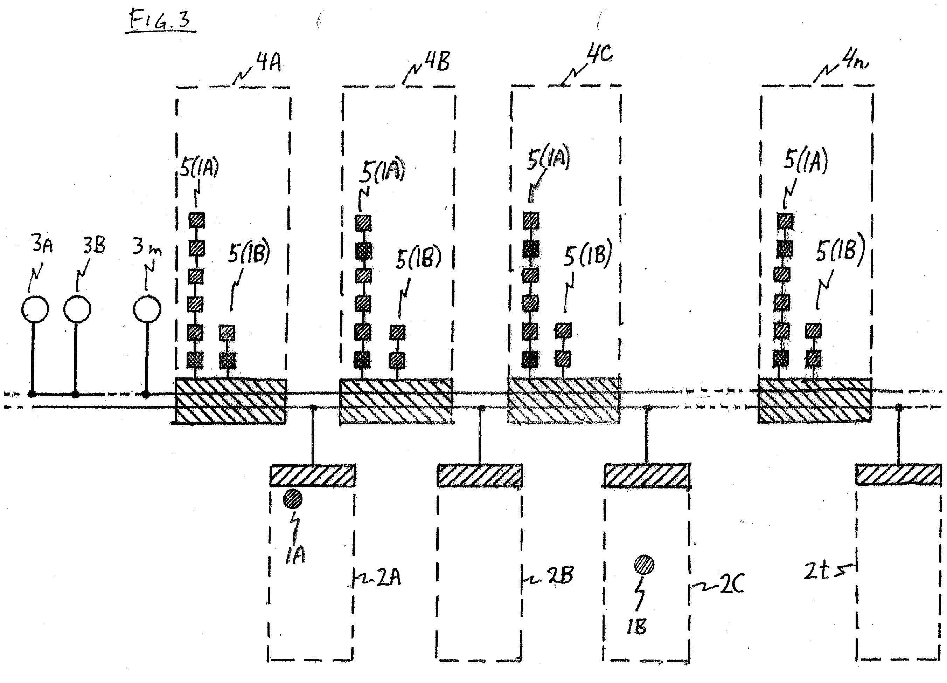

As shown in **Fig.1****,** the biobank and database system involves interactions between three types of physical entities, i.e. the node (**4**), the incubator (**2**) and the operator (**3**). These shall typically be located remote from each other and communicate via secure data lines. This architecture opens up for a distributed collaborative network as illustrated in **Figs.2****,** **3****:** In **Fig.2** the node (**4A**) is linked with a set of parallel nodes **(4B),(4C)..(4n),** which are all functionally similar and on an equal footing regarding contents and input/output operations. Thus, a blockchain in one node is mirrored in all nodes and any addition to a blockchain in one of the nodes is instantly added to the blockchains in all other nodes. The nodes are linked via a common data channel (**10**) which also connects to a community of operators (**3A**), (**3B**),..(**3m**). The operators, e.g. researchers, access the system on an equal footing via the common data channel (**10**). A plurality of incubators (**2A**), (**2B**), (**2C**),..(**2t**) are accessible to the system via the data channel (**11**) which links in parallel to all nodes (**4A**)-(**4n**). In the example shown in **Fig.2****,** only one sample (**1A**) is stored in one of the incubators (**2A**). All information pertaining to this sample is described in a blockchain (**5(1A)**) which is copied equally in all nodes **(4A)-(4n).** An operator who wishes to perform an operation affecting the sample (**1A**) must first log onto the network via the data channel (**10**), identifying himself and supplying information on the location of the sample (**1A**) that he wishes to access and the operation in question. The incubator (**2A**) shall be accessed via one of the nodes, in this case (**4A**), where the interface (**8A**) acts as a gate-keeper and communications hub for the specific operation in question. Once the operation is completed, this is reported back via the incubator and node interfaces (**9A**), (**8A**). The node (**4A**) prepares a block and adds it to the chain (**5(1A)**), and the change is immediately updated in all nodes (**4A**)-(**4n**) in the network.

**Fig.3** shows the system at a later point in time. The blockchain (**5(1A)**) has now received two new blocks, and another biological sample (**1B**) in incubator (**2C**) has been introduced. The latter is reflected in the blockchain (**5(1B)**) which is established along with the old one in all nodes. The system architecture allows the number of samples and blockchains to be expanded virtually without limit, and new operators, nodes and incubators can be included overtime without disrupting operations or requiring retroactive changes of the system.

The architecture shown in **Figs.2****,****3** provides opportunities for a large range of collaboration models in research and product development: Each blockchain represents a sequence of operations on a specific sample in an incubator and is typically open to review by all operators in the system. However, any new operations on the sample shall be contingent on the operator in question having clearance to access this particular sample. This shall typically be the case for the operator who initiated research on the sample, but may also be granted to other operators who can continue doing research on the sample and add elements to the blockchain in question.

Often, this shall be undesirable, since the sample may be irreversibly changed. An alternative mode of pursuing a specific line of research based on information contained in a given blockchain is to establish a new parallel sample in an incubator, where all physical attributes pertaining to the original sample, as well as the operations that have been performed on it up to a given point in the original blockchain are copied and recorded in a new blockchain. The operator creating this new blockchain shall not only be free to pursue further research on the new sample without interfering with the old one, but he shall also have the option of backstepping and starting his research at a point earlier in the chain where the last operations in the old chain are exchanged for new and alternative operations on the sample. This procedure can in principle be applied any number of times, where the operator establishes parallel samples and associated blockchains, enabling the operator to test out different procedures and materials from a common starting point. Establishing a family of blockchains from a common base does not break with the blockchain principle, since the original blockchain remains intact, as do all the new blockchains established in this way.

According to the present invention, a network of nodes form a distributed peer-to-peer blockchain system. There is no centralized master node, and information is updated in parallel in all nodes. In the present case, the network is only accessible to a limited set of participants that form a consortium. Other types of networks are possible, e.g. a combination of public and private networks that allow access for a wider public to read the content of blockchains whereas adding to the blockchains is restricted to a private consortium. A mechanism for safeguarding the system against falsifying the blockchain record is to enforce distributed consensus within the consortium where all nodes in the system use identical history of data to decide which blockchain that represents the truth. Many different consensus algorithms exist, with the goal of ensuring that "... the next block in a blockchain is the one and only version of the truth" (Castor, A.: "A (Short) Guide to Blockchain Consensus Protocols". (Online) https://www.coindesk.com/short-guide-blockchain-consensus-protocol/).

The distributed blockchain architecture allows unlimited expansion by addition of new nodes. Each new node must be accepted by the existing peer network and assumes responsibilities for exercising all protocols regarding access for and communication with individual operators.

As is clear from the description above, the present invention enables an unprecedented level of interactivity between operators from separate institutions at sites that may be remote from each other working on a specific research task, while at the same time protecting and preserving the work performed by each operator. Backward compatibility and reference frames are vital, and an essential feature of the present invention is a requirement of strict adherence to a common protocol to ensure that all aspects of the research and communication are compatible and harmonized between all parties involved. This must apply to all phases of the physical operations as well as nomenclature and formats for data that are stored in the blockchain, and shall include, e.g., detailed procedures for cell harvesting, sample preparation, printing, preservation and storage in the incubators, possibly extending to the specific equipment used. As an example, it may be agreed to employ a given brand and model of 3D printer in certain lines of inquiry.

The biobank and database system according to the present invention provides solutions to some of the most important concerns facing individual researchers, firms and institutions that engage in ex vivo research on living tissue for diagnostics, therapy, drug development, drug testing, regenerative medicine and tissue engineering, biocompatibility testing, etc:
- Priority: Establishing priority is important for scientific acknowledgement and for the reputation of the individual researcher as well as for his or her parent institution. It may also have economic impact, e.g. in establishing "first to invent" precedence in IPR conflicts. Traditionally, priority has been established through publication in journals or patent applications, but these modalities are often subject to long delays and associated risks. The present invention provides a virtually instantaneous, time-stamped and secure record of results that are linked to the operator in question.
- Cooperation, interactivity, speed: By essentially removing potential conflicts relating to priority issues, the scientific discourse can take place in a more relaxed and open atmosphere which is conducive to cooperation between different research groups. Coupled with near-instantaneous communication of results, the R&D process can be greatly accelerated. The organhotel concept is particularly well adapted to handle parallel investigations on a large number of samples from a common starting point, further contributing to speedy R&D progression.
- Searchability and analysis: The blockchain system according to the present invention makes the stored information directly accessible to a large community of operators with different skills and analytic resources, in a standardized format which can support various types of search queries. Thus, the information can be subjected to a vastly more varied and comprehensive analytic process than would be within the capabilities of a single R&D group, revealing hidden relationships and promoting a deeper understanding of the complex processes involved.

The biobank and database system as taught in the present invention is ideally suited for automated research operations under Artificial Intelligence (Al) guidance. On an elementary level this could include large scale screening of organoid responses to medicines, toxic substance, growth factors, etc, where operations are defined and initiated by specific requests from external agencies such as researchers or donors of biobank samples. However, operations may also be defined and initiated by feedback from monitoring of ongoing activities within the system itself, where AI algorithms identify suitable conditions for initiating new research and define guidelines for this research. As an example, new lines of research may be based on analysis of sensor returns describing the state of certain samples in the biobank, indicating that trigger levels for certain parameters have been exceeded. The sensors may be of a non-contact type, e.g. incubator based observation equipment providing imaging or spectroscopic data, or they may be contacting the organoids or tissue samples directly, to provide information not accessible otherwise. The latter is particularly relevant in view of current research on miniaturized bioelectronic devices that combine sensor functions with communication. An example of this are micro RFID chips that naturally integrate into the cells of developing organoids to record any interesting changes as the organoids grow (see, e.g. M. Kimura et al.: Digitized Human Organoid for Wireless Phenotyping" iScience 4, 294-301, June 29, 2018; https://www.cell.com/action/showPdf?pii=S2589-0042%2818%2930062-2).

Implanted miniaturized bioelectronic devices are expected to play an important role not only in diagnostics and health monitoring in the future, but also therapeutically. An example of the latter is research currently underway to develop bioelectronic medicines designed to treat disease by modulating electrical signals in peripheral nerves using miniature, implanted devices This may provide an entirely new toolkit for control and reversal of disease, and could complement pharmaceutical and other therapies (see, e.g. Galvani Bioelectronics: https://verily.com/projects/interventions/galvani-bioelectronics/).

These examples provide a glimpse of the vast array of novel in vitro research opportunities linked to device development and testing. More specifically, they highlight the urgent need for biobanks and database systems as taught in the present invention that are capable of providing high throughput, automated, secure and intelligent research, testing and qualification of materials, devices and procedures in the future.

## Claims

1. Biobank and database system for storage of and interactions with biological samples in vitro, comprising:
- incubators for living tissue;
- a number of biological samples, where each biological sample is either an organoid or a sample containing live tissue, supported by or containing cellulose derived from tunicates, and arranged in one or more of the incubators;
- means for monitoring each biological sample;
- means for manipulating each biological sample;
- a chronologic ledger with automatic registration of data pertaining to:
provenance and pre-history of each biological sample, including materials and procedures employed in extraction and preparation;
manipulations performed on each biological sample; and results obtained;
- one or more nodes arranged to communicate with the incubators, and control access to the chronologic ledger and to the biological samples in the incubators, each node comprising a logic unit, a data storage facility, and a communication interface.

2. Biobank and database system according to claim 1, where the cellulose derived from tunicates is tunicate nanocellulose.

3. Biobank and database system according to claim 1 or 2, where the biological samples in the incubators are formed by one or more of the following techniques: 3D bioprinting, micro-contact printing, nano-imprint lithography, selective etching, and electrospinning.

4. Biobank and database system according to one of the claims above, where the means for manipulating are robotic.

5. Biobank and database system according to one of the claims above, where the chronologic ledger is arranged for performing automatic registration of accesses to at least one of the biological samples, including accesses for purely monitoring purposes.

6. Biobank and database system according to claim 5, where the chronologic ledger is searchable.

7. Biobank and database system according to one of the claims 5 or 6, where two or more of the nodes form a communicating network operated under a consensus protocol.

8. Biobank and database system according to claim 7, where the communicating network is only accessible to a limited set of participants that form a consortium.

9. Biobank and database system according to claim 8, where subsets of consortium participants can be formed when conforming with the consensus protocol.

10. Biobank and database system according to claim 8 or 9, where each node is arranged for regulating network access for individual research entities associated with that node.

11. Biobank and database system according to claim 8 to 10, where the chronologic ledger is arranged for being globally accessible for searches, including to parties outside the consortium, under a protocol where each search query incurs a fee.

12. The biobank and database system according to one of the claims above, where the chronologic ledger is structured as a blockchain.

13. The biobank and database system according to one or more of the claims above, where individual biological samples are monitored by microsensors that transmit data to the chronologic ledger.

14. Biobank and database system according to one or more of the claims above, where the biobank and database system is operated in an autonomous or semi-autonomous mode under Artificial Intelligence (Al) guidance.

15. Biobank and database system according to one or more of the claims above, where the biobank and database system is operated for screening tests of at least one from the group comprising: medicines, allergens, toxic chemicals, growth factors, chemical additives in foodstuff and consumer products.

16. Method for operating a biobank and database system according to one of the preceding claims, where the method comprises at least one of the following steps:
- i) Depositing one or more organoids and/or tissue samples that are supported by or contain cellulose derived from tunicates, at specific locations in one or more incubators;
- ii) bringing the organoids and/or tissue samples in contact with a test substance;
- iii) monitoring the organoids and/or tissue samples; and
- iv) recording data from events related to the organoids and/or tissue samples in a chronologic ledger organized as a blockchain.

17. Method for operating a biobank and database system according to claim 16, where the test substance is a foodstuff or a therapeutic substance.

18. Method for operating a biobank and database system according to one of the claims 16 and 17, where the deposition of one or more organoids and/or tissue samples is performed by 3D printing or by transporting a pre-made organoid or tissue sample to the biobank.
